# FASCICULE DE BREVET EUROPEEN

(11) **EP 1 165 134 B1**
(45) Date de publication et mention de la délivrance du brevet: **16.03.2005**
(21) Numéro de dépôt: 01907655.3
(22) Date de dépôt: 19.01.2001
(51) Int. Cl.: A61K 39/395, G01N 33/574, G01N 33/577, G01N 33/58, A61K 47/48, A61K 51/10, A61P 35/00

(54) **UTILISATION D'ANTICORPS MONOCLONAUX ANTI-FERRITINES DANS LE TRAITEMENT DE CERTAINS CANCERS**
VERWENDUNG VON ANTI-FERRITIN ANTIKÖRPER BEI DER BEHANDLUNG VON BESTIMMTEN KREBSARTEN
USE OF ANTI-FERRITIN MONOCLONAL ANTIBODIES IN THE TREATMENT OF SOME CANCERS

(30) Priorité: 20.01.2000 FR 0000718
(43) Date de publication de la demande: 02.01.2002
(73) Titulaire: Monoclonal Antibodies Therapeutics M.A.T., 75013 Paris (FR)
(72) Inventeur: KADOUCHE, Jean, F-75013 Paris (FR); LEVY, Rafael, F-92120 Montrouge (FR)
(74) Mandataire: Desaix, Anne
(86) Numéro de dépôt international: PCT/FR2001/000192
(87) Numéro de publication internationale: WO 2001/052889

(56) Documents cités:
- US-A- 5 538 726
- US-A- 5 843 440
- ONO K ET AL: "Common epitopes in human isoferritins characterized by murine monoclonal antibodies." JOURNAL OF BIOCHEMISTRY, (1986 JAN) 99 (1) 269-79., XP000930082
- JEANTEUR P: "[c- Myc: an iron oncogene (news)]. c- Myc: un oncogene de fer." BULLETIN DU CANCER, (1999 MAR) 86 (3) 250., XP000930066
- HANN H W ET AL: "Basic and acidic isoferritins in the sera of patients with neuroblastoma." CANCER, (1988 SEP 15) 62 (6) 1179-82., XP000930056
- PEARCE L A ET AL: "Linear gene fusions of antibody fragments with streptavidin can be linked to biotin labelled secondary molecules to form bispecific reagents" BIOCHEMISTRY AND MOLECULAR BIOLOGY INTERNATIONAL,ACADEMIC PRESS, LONDON,GB, vol. 42, no. 6, septembre 1997 (1997-09), pages 1179-1188, XP000964689 ISSN: 1039-9712
- DATABASE BIOSIS [en ligne] BIOSCIENCES INFORMATION SERVICE, PHILADELPHIA, PA, US; 1982 KADOUCHE J ET AL: "Analysis of various iso ferritins with mono clonal antibodies" Database accession no. PREV198376041611 XP002168302 & COMPTES RENDUS DES SEANCES DE L'ACADEMIE DES SCIENCES SERIE III, vol. 295, no. 6, 1982, pages 443-448, 1982 (RECD. 1983) ISSN: 0249-6313
- VRIESENDORP ET AL CANCER vol. SUP. 80, no. 12, 1997, pages 2721 - 2727
- WU ET AL: 'Coordinated Regulation of Iron-Controlling Genes H-Ferritin and IRP2, by c-MYC' SCIENCE vol. 283, 1999, pages 676 - 679

## Description

La présente invention vise à fournir de nouveaux moyens pour le diagnostic et la thérapie des cancers caractérisés par une sur-expression des produits des gènes de la famille Myc. Ces moyens incluent essentiellement l'utilisation d'anticorps monoclonaux reconnaissant un épitope commun aux isoferritines acides et basiques.

La ferritine est une protéine d'un poids moléculaire de 440.000 kda utilisée pour le stockage du fer dans les cellules. Depuis 1942, date à laquelle GRANICK isola la ferritine hépatique et jusqu'à ces dernières années, cette molécule était considérée comme une protéine de stockage du fer, uniquement cellulaire, principalement localisée dans le foie, la rate et la moelle osseuse. Elle semblait n'apparaître dans le sérum qu'à l'occasion d'une surcharge ferrique importante ou lors d'une nécrose hépatique avec libération de cette protéine tissulaire dans l'espace vasculaire. Des méthodes de détection plus sensibles réalisées à l'aide de traceurs radioactifs, enzymatiques ou fluorescents ont permis de mettre en évidence l'existence d'une concentration basale en ferritine sérique et d'étudier les variations de celle-ci au cours de diverses pathologies. Parallèlement à ces travaux, quelques équipes de recherche se sont efforcées :
- d'affiner les connaissances relatives à la structure biochimique de la ferritine tissulaire ;
- de préciser la signification de l'hétérogénéité des formes moléculaires désignées sous le terme "d'isoferritines" ;
- de comparer les différentes caractéristiques de la ferritine extraite du plasma ou de divers organes sains ou pathologiques.

Les données actuelles montrent que la ferritine est un édifice macromoléculaire constitué d'une structure glycoprotéique en forme de coque, l'Apoferrifine, qui en est le composant protéique et qui renferme au sein de la cavité qu'elle constitue un noyau métallique d'atomes de fer distribués en cristaux ferriques

L'analyse cristallographique aux rayons X de la ferritine a révélé la présence de 24 sous-unités, distribuées selon une symétrie rigoureuse. Les sous-unités sont de deux types H et L.

Les gènes codant pour chacune de ces sous-unités ont été clonés et leur localisation chromosomique est connue (Murow H.N., Aziz N., Leibold E.A. et al, The Ferritin gènes expression and regulation, Ann NY Acad. Sci. 1988, 528 113). La sous-unité de type H, de poids moléculaire 21 000 est majoritaire parmi les isoferritines les plus acides telle la ferritine extraite des cellules HeLa ou la ferritine d'origine cardiaque (H = heart) mais elle est présente également en moins grande quantité dans le foie et la rate. Le deuxième type de sous-unité (L) de poids moléculaire 16 000 est majoritaire de façon plus spécifique parmi les molécules les plus basiques d'origine hépatique (L = liver) ou splénique.

Le fer stocké dans la ferritine est mobilisé pour la synthèse de protéines qui incorporent des ions ferreux ou ferriques comme composants fonctionnels (hémoglobines et enzymes telles les cytochromes et les catalases). Les principales fonctions de la ferritine dans le foie, la rate et la moelle sont, d'une part, de protéger les tissus de l'oxydation et des dommages causés par les radicaux libres produits par les interactions avec le fer, l'eau et l'oxygène et, d'autre part, de réutiliser le fer pour la synthèse d'hémoglobines. Dans les autres organes ou dans le plasma, la ferritine ne contient pas ou peu de fer et sa fonction est actuellement mal comprise.

Il est connu depuis longtemps qu'il existe différents phénotypes de ferritine (isoferritines) (Drysdale J.W. (1977), Structure and metabolism, (Ciba Foundation Symposium 51 New series), pages 41-57. Dès 1965, Richter démontra que la ferritine préparée à partir de foie humain et de cellules carcinomateuses épidermoïdes HeLa présentait des vitesses de migration électrophorétique différentes sans que les différences de contenu en fer puissent l'expliquer. Au total, une vingtaine d'isoferritines ont été
caractérisées.

Par des techniques comme l'électrofocalisation, la chromatographie échangeuse d'ions, l'électrophorèse bidimensionnelle en conditions dénaturantes, on a montré que les isoferritines sont formées par la combinaison variables en proportion des deux sous-unités H et L (Murow et al, supra).

Le rôle des isoferritines basiques est bien connu, particulièrement celui de la ferritine sérique dans les phénomènes directement liés au métabolisme du fer. Celui des ferritines acides est controversé : de nombreux auteurs en font un marqueur tumoral chez l'homme. Dès 1968, il a été décrit une α2 H globuline, plus tard dénommée α2 H isoferritine, présente dans le sérum de malades atteints de différentes néoplasies (Buffe D. et al, Présence d'une protéine d'origine tissulaire α2-Hglobuline dans le serum de sujets atteints d'affections malignes, Int. J. Cancer (1968) 3: 850-856). Marcus D. et al (J. Natl. Cancer Disti. (1975) 55:791.-795) décrivent à leur tour une ferritine anormale dans le sérum de malades atteints de cancer du sein. Drysdale J. et al, (Cancer Res. (1974) 34: 3352-3361) décrivent une isoferritine acide identique à la ferritine contenue dans les cellules HeLa et dont la concentration est particulièrement élevée dans différents types de néoplasies. Moroz C., et al. (Clinical Exp. Immunol. (1977) 29: 30-76) décrivent un facteur, qui est une isoferritine, sécrété par une sous-population de lymphocytes T présents particulièrement dans le cancer du sein et la maladie de Hodgkin. Il est notoire enfin que la plupart des processus néoplasiques humains (Maladie de Hodgkin, cancers du sein, de l'ovaire, du pancréas, du poumon, cancers épidermoïdes de la tête et du cou, neuroblastomes, leucémies aiguës lymphoblastiques (LAL), sarcomes de Kaposi etc...) s'accompagnent d'une élévation du taux de ferritine sérique. Dans tous ces cas, un niveau de ferritine sérique élevé est un facteur pronostique négatif (Hann H.W., Evans A.E., Siegel S.E. et al, Cancer Res., (1985) 45: 2843-2848 ; Jacobs A., Slater A. Wittaker J.A. et al., J. Cancer, (1976) 34: 533).

Il a pu être montré par ailleurs dans quelques types de cancers une augmentation locale tumorale de la ferritine tissulaire, sans que l'origine réactionnelle stromale ou purement tumorale de cet excès de ferritine ait été formellement précisée. Néanmoins, tous les auteurs sont d'accord pour considérer qu'il s'agit de ferritine acide.

Le développement des anticorps, et notamment des anticorps monoclonaux, dans l'imagerie ou pour le traitement de certaines pathologies a fait l'objet de très nombreux développements.

Une revue de ces développements a été publiée en 1998 (P.S. Multani et al. (1998), Journal of Clinical Oncology, vol. 11, 16: 3691-3710). L'utilisation diagnostique ou thérapeutique des anticorps dans un système de ciblage de cellules avec des substances ayant une toxicité sélective pour éliminer spécifiquement des cellules pathologiques est connue sous le nom anglo-saxon de "magic bullet".

Les différentes possibilités d'utilisation des anticorps monoclonaux dans ce concept sont schématiquement les suivants :
a) l'utilisation de l'anticorps natif comme effecteur immunitaire, faisant appel dans les thérapies anti-tumorales à une activité CDCC (pour complément dépendent cell cytotoxicity) ou ADCC (antibody dépendant cell cytotoxicity). L'anticorps présentant une certaine spécificité vis-à-vis de cette cellule cible via ses fragments Fab peut ensuite entraîner une réaction d'immunité cellulaire grâce aux fragments Fc du même anticorps. Il peut s'agir aussi d'un blocage d'un récepteur de la cellule cible ou d'une vaccination anti-idiotypique spécifique de l'antigène tumoral ;
b) l'anticorps peut être couplé à une toxine ou à un médicament ; l'anticorps est alors un vecteur qui transporte ladite toxine ou ledit médicament sur la cible ;
c) les anticorps peuvent également jouer un rôle de vecteur galénique par couplage avec des liposomes ou d'autres systèmes analogues dans lesquels peuvent être introduits des substances cytotoxiques ou des médicaments, soit des oligonucléotides anti-sens, etc.
d) les anticorps peuvent être radiomarqués directement ou indirectement (chelate ou anticorps bispécifique avec site «antichelate, etc.») et leur rayonnement utilisé à des fins thérapeutiques ou d'imagerie. Parmi les isotopes utilisés, notamment pour marquer les anticorps, plusieurs possibilités existent. La plus généralement utilisée fait appel à l'iode 131. Cette méthode a de nombreux inconvénients (fixation de l'iode sur la thyroïde, existence de déshalogénases endogènes). De plus, l'iode 131 n'émet pas un rayonnement β pur. Enfin, sa distance d'irradiation est courte (1,1 mm).

Le couplage à l'indium 111 pour l'imagerie ou l'Yttrium 90 pour la thérapie a été également développé. Dans ce cas, le couplage est généralement réalisé par greffage d'un chelate sur l'anticorps, le chelate fixant l'émetteur radioactif selon des techniques décrites et développées par l'équipe de S.M. Quadri et H.M. Vriesendorp (Vriesendorp H.M. et al. (1991), J. Clin. Oncol. 9: 918-928 ; P.E. Borchardt et al. (1998), The Journal of Nuclear Medicine 39: 476-484). Le choix de l'Yttrium 90 comme radio-isotope thérapeutique est pertinent pour la cancérologie. C'est en effet un isotope qui émet un rayonnement β pur ; sa demi-vie est de 67 heures et son rayon d'irradiation est de 6,6 mm. Il semble ainsi bien adapté au traitement des masses tumorales solides, notamment la maladie de Hodgkin et les lymphomes malins non hodgkiniens. Des résultats encourageants ont été obtenus par la même équipe (H.M. Vriesendorp et al. (1995), Cancer Research, 55: 58-92). Ces auteurs décrivent des essais de traitement avec des anticorps polyclonaux anti-ferritine marqués à l'Y 90 chez des patients ayant une maladie de Hodgkin résistante aux méthodes de traitement classiques associant chimiothérapie et radiothérapie externe et greffes de moelle osseuse.

Effectivement, une des caractéristiques biologiques de cette maladie est une hyperexpression par les cellules tumorales et les cellules réactionnelles qui les entourent de ferritines. L'imagerie des sites tumoraux (Indium 111) et le traitement des formes résistantes de cette maladie par le système décrit dans Vriesendorp et al (1995), Cancer Research, 55: 58-92 a donné de bons résultats.

Par ailleurs, il est connu que la famille d'oncogènes Myc (pour les plus importants c, N, L) code pour des protéines nucléaires se liant à l'ADN. Dans une cellule normale, la transcription des gènes Myc augmente dans les heures suivant un signal mitogénique. Les protéines codées par les gènes de la famille myc possèdent un motif "leucine zipper" (fermeture à glissière leucine). Cette structure permet la formation d'hétérodimères (avec c-fos ou c-jun) ou d'homodimères. On conçoit actuellement la fonction du produit de c-myc comme une fonction de régulation de la transcription de gènes cellulaires impliqués dans l'initiation de la mitose. De façon schématique, les gènes myc acquièrent des propriétés oncogènes par des mécanismes qui aboutissent à une surexpression de leurs produits. Cette surexpression peut être secondaire :
- soit à une amplification du gène,
- soit à une activation de la transcription du gène,
- soit à des modifications post-transcriptionnelles entraînant une stabilité accrue des ARNm. L'étude *in vivo*, de la surexpression de produits de gènes de la famille myc dans différents tissus par la création d'animaux transgéniques, confirme ces faits. Enfin toute augmentation de prolifération s'accompagne physiologiquement d'une expression accrue de gènes de la famille myc.

Les trois membres principaux de la famille myc (c, N, L) sont parmi les oncogènes les plus fréquemment retrouvés activés, par ces trois différents mécanismes, dans les cancers humains. En particulier, c-myc dans les lymphomes malins, L-myc et N-myc dans les cancers du poumon à petites cellules et les neuroblastomes d'où ce dernier a été caractérisé. Dans la plupart des tumeurs malignes humaines, le degré d'expression des gènes myc est un facteur pronostique négatif.

Les gènes cibles connus dont la transcription est régulée par les produits des gènes de la famille myc sont peu nombreux. Le gène codant pour la chaîne H de la ferritine est le dernier gène dont il vient d'être montré qu'il était régulé négativement par le produit du gène c-myc (Wu et al., Coordinated regulation of iron controlling gènes, H ferritin and IRP2 c-myc. *Science* (1999) 283 : 676-679). Autrement dit, l'expression du gène c-myc et/ou d'autres membres de cette famille dans les cellules cancéreuses est inversement proportionnelle à l'expression de la ferritine.

La présente invention résulte de la constatation inattendue et non prévisible d'après les données disponibles exposées ci-dessus selon laquelle certains anticorps monoclonaux après immunisation de souris avec une ferritine extraite et purifiée à partir d'une rate humaine présentaient une très grande spécificité dans le ciblage de cellules cancéreuses ou de cancers dans lesquels le gène myc est sur-exprimé.

La technique d'extraction-purification de la ferritine utilisée permet d'obtenir les deux formes acide et basique de la ferritine avec un grand taux de pureté. La technique d'extraction-purification mise en oeuvre dans la présente invention consiste en particulier à l'extraction et la purification de ferritines humaines extraites et purifiées de patients atteints d'anémie microsphérocytaire. De façon inattendue certains des anticorps monoclonaux, obtenus par utilisation de la technique classique de Kölher et Milstein, reconnaissent un épitope commun à toutes les isoferritines acides et basiques et uniquement humaines (J. Kadouche et al C.R. Acad. Sc. Paris (1982) t. 295 série III, page 443). Le screening des anticorps monoclonaux se fait en deux étapes : i) sélection des anticorps antiferritine par capture sur phase solide recouverte avec la même ferritine que celle utilisée pour l'immunisation des animaux ; ii) révélation de la fixation des anticorps spécifiques anti-ferritine fixés sur cette phase solide par ajout dans les puits de la même ferritine radiomarquée à l'iode 125. La sélection des anticorps sur phase solide à l'aide de la ferritine purifiée et en utilisant la ferritine marquée à l'iode 125 permet ainsi de sélectionner des anticorps dirigés contre un épitope commun et répétitif à toutes les isoferritines acides et basiques humaines. On entend par « épitope répétitif », un même épitope présent en plusieurs exemplaires sur une molécule.

L'invention résulte également de la mise en évidence selon laquelle, contrairement à ce qui était prévisible au vu de l'article de Wu et al. (supra), les anticorps monoclonaux spécifiques des deux formes de ferritine reconnaissent les cellules tumorales ou les tissus cancéreux dans lesquels le gène C-myc est surexprimé.

La présente invention porte sur l'utilisation, dans la fabrication d'un médicament pour la thérapie d'un cancer dont les cellules présentent une surexpression du produit d'un gène de la famille c-myc, L-myc et N-myc, d'un anticorps monoclonal anti-ferritine ou un fragment de celui-ci ou d'une construction incluant un tel fragment, ledit anticorps ou ledit fragment reconnaissant un épitope commun aux ferritines humaines acides et basiques.

Par construction incluant un fragment d'anticorps, on entend un produit d'une séquence polynucléotidique codant ledit fragment et un polypeptide présentant une fonction de stabilisation ou de transport, tei l'albumine, l'ovalbumine ou un fragment de celles-ci. On peut également entendre un conjugué associant l'anticorps ou le fragment d'anticorps à un adjuvant et/ou une molécule ou structure assurant le transport et/ou la stabilité dudit anticorps, comme des liposomes, des vésicules cationiques, des nanoparticules. Il peut s'agir enfin d'une combinaison du produit d'une séquence telle que décrite ci-dessus conjugué à un adjuvant et/ou une molécule ou structure assurant le transport et/ou la stabilisation de la molécule.

Une utilisation thérapeutique ou diagnostique in vivo des anticorps monoclonaux nécessite que ceux-ci réunissent des qualités particulières de spécificité, d'affinité et de non-toxicité. Par non-toxicité, on entend le fait que ceux-ci n'induisent pas de réactions immunitaires ou de rejets vis-à-vis d'une substance susceptible d'être administrée à long terme et de toutes les façons en administration répétée. Par spécificité, on entend le fait que les anticorps monoclonaux en cause ne présentent pas de réaction croisée avec des antigènes autres que les ferritines.

En terme de spécificité, il est important que l'anticorps reconnaisse à la fois les isoferritines acides (spécifiques des processus néoplasiques, forme H) et les isoferritines basiques (métabolisme martial forme L).

L'anticorps monoclonal antiferritine selon l'invention doit également avoir une affinité pour l'antigène correspondant suffisante de façon à éliminer au maximum la diffusion de cet anticorps, le cas échéant porteur d'une substance toxique ou thérapeutique dans des tissus ou des cellules sains. Dans le cadre de la présente invention, l'affinité pour les ferritines doit être supérieure à 10⁻⁹ Mole/litre.

Par ailleurs, il est préférable que l'épitope commun aux isoferritines acides et basiques soit un épitope répétitif. La répétition d'un même épitope sur les isoferritines permet la fixation de plusieurs anticorps sur une même molécule de ferritine. Les propriétés diagnostiques et thérapeutiques des produits de couplage résultants de ces anticorps sont ainsi avantageusement améliorées.

L'invention porté également sur l'utilisation des anticorps monoclonaux antiferritines pour le diagnostic ou la thérapie des cancers ou des cellules tumorales dans lesquels une surexpression d'un gène de la famille c-myc, L-myc et N-myc est observée et qui présentent les caractères suivants :
- il reconnaît un épitope commun aux ferritines acides et basiques et, de préférence, il ne reconnaît pas les ferritines non humaines et mieux encore, ledit épitope commun aux ferritines acides et basiques est un épitope répétitif;
- son affinité pour l'antigène est supérieure à 10⁻⁹ Mole/litre.

Les anticorps utilisés selon l'invention sont de préférence des immunoglobulines IgG de type Kappa. Mais, il peut également s'agir d'IgM qui, par la multiplicité de leurs sites de reconnaissance épitopique, permet d'améliorer le ciblage. Lorsque l'on effectue une injection in vivo directement dans la lésion, l'IgM est préférée dans la mesure où elle a dix sites de liaison à l'antigène ; le rendement est alors meilleur.

Par diagnostic, on entend tant le diagnostic in vitro que l'immunoscintigraphie ou toute méthode de diagnostic in vivo utilisant l'anticorps monoclonal comme vecteur d'une substance permettant sa localisation.

La qualité des anticorps monoclonaux utilisables selon l'invention résulte, d'une part, de la qualité de l'antigène utilisé pour l'immunisation et, d'autre part, de son degré de pureté.

La ferritine utilisée pour immuniser les souris dans le procédé d'obtention des anticorps monoclonaux provient d'une rate pathologique d'une maladie de Minkowski-Chauffard extraite par la méthode de Granick au sulfate d'ammonium et au sulfate de cadmium modifiée. C'est cette ferritine extraite et purifiée qui, selon un protocole décrit dans l'exemple 1 ci-après qui a été utilisée pour immuniser les souris afin d'obtenir les hybridomes producteurs des anticorps monoclonaux.

Le tableau I ci-dessous résume un certain nombre de caractéristiques des anticorps obtenus après clonage des hybridomes obtenus selon la technique d'extraction purification et immunisation selon l'invention.

Dans ce tableau, sont indiquées la dénomination des monoclonaux et des polyclonaux testés, leur nature isotypique, leur affinité vis-à-vis de la ferritine et la réactivité vis-à-vis de toute une série de ferritines mesurée par précipitation par la technique d'immuno-diffusion d'Ouchterlony. Les anticorps utilisables selon l'invention doivent avoir une réactivité négative pour toutes les cellules ou tissus non humains, à savoir la rate de cheval et la LIA (pour le lemia inhibitors activities), ce qui n'est le cas pour aucun des anticorps polyclonaux ni pour le monoclonal M211.

Une autre caractéristique des anticorps utilisables selon l'invention est leur constante d'affinité ; il apparaît que parmi les anticorps de ce tableau, seuls le B8 et M29 sont utilisables dans l'invention.

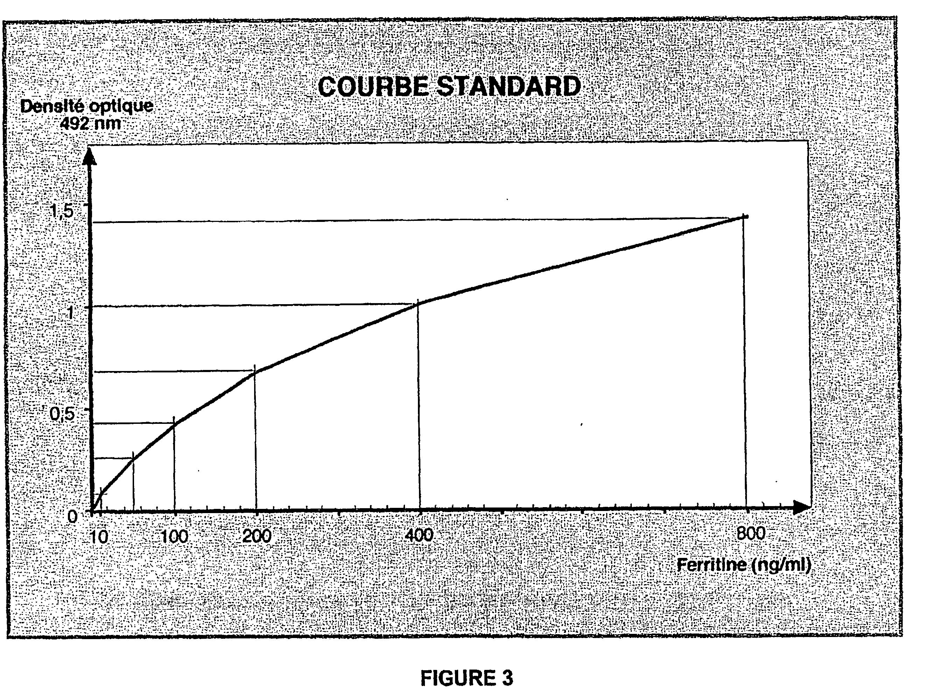

Enfin, dans un mode de réalisation particulier, les anticorps utilisés selon l'invention sont dirigés contre un épitope répétitif commun aux isoferritines acides et basiques. De tels anticorps sont mis en évidence à l'aide d'un test Sandwich ELISA tel que décrit dans l'exemple 2 ci-après et illustré à la figure 3. La possibilité de mise en oeuvre d'un tel test confirme que plusieurs anticorps peuvent se fixer sur une même molécule de ferritine. Cette propriété confère un avantage déterminant pour leurs utilisations diagnostique ou thérapeutique.

| | | | | | | | |
|---|---|---|---|---|---|---|---|
| Anticorps monoclonal | M29 | M211 | M386 | B8 | - | - | - |
| Anticorps polyclonal | - | - | - | - | F1 | F2 | F3 |
| Isotypes | Ig G1K | Ig G1 K | Ig G2b K | Ig G1 K | - | - | - |
| Constante d'affinité (L/Mol) | 2.3 10⁻¹⁰ | 1.3 10⁻⁹ | 2.2 10⁻⁸ | 5.1 10⁻⁹ | 1.3 10⁻¹¹ | 5.9 10⁻¹¹ | 4.0 10⁻¹¹ |
| Réactivité : | | | | | | | |
| . foie humain | + | + | + | + | + | + | + |
| . rate humaine | + | + | + | + | + | + | + |
| . coeur humain | + | + | + | + | + | + | + |
| . placenta humain | + | + | + | + | + | + | + |
| . cellules HeLa | + | + | + | + | + | + | + |
| . rate de cheval | - | + | - | - | - | + | + |
| . LIA | - | - | - | - | + | + | + |

L'usage in vivo d'anticorps monoclonaux murins ou animaux est néanmoins limité du fait de la production par l'organisme d'anticorps anti-espèces rendant rapidement inefficaces voire dangereuses (anaphylaxie, maladie à immuns-complexes, production d'anticorps anti-idiotypes) les injections itératives. Il est donc préférable d'éliminer les parties Fc de ces anticorps murins, soit par humanisation, soit par reconstitution. La solution consistant à développer des anticorps chimériques a souvent été utilisée (Nature 321 (1986) 321: 522-525). Il est désormais possible, de cloner les gènes codant pour les deux chaînes des anticorps monoclonaux produits par des hybridomes, ceux-ci pouvant être manipulés in vitro et réintroduits ensuite dans des cellules secrétrices, telles des lignées lymphoïdes ou autres, après leur réinsertion dans les vecteurs d'expression appropriés aux cellules choisies. De nouveaux anticorps monoclonaux sont ainsi construits avec des régions variables de souris ou de rats et des régions constantes humaines. Différents types d'immunoglobulines peuvent être obtenus. Leur spécificité dans tous les cas sera la même que celle des régions variables utilisées mais ils posséderont les propriétés effectrices des immunoglobulines humaines suivant l'isotype choisi dans la construction.

Une seconde approche consiste à reconstituer les anticorps (Reshape antibodies) (Riechmann et al. (1988), Nature 332: 323-327). Schématiquement, le point de départ de cette approche est l'observation que les sites de contact des anticorps avec les épitopes des antigènes qu'ils reconnaissent sont localisés sur certaines séquences des régions variables dites hypervariables. La technique consiste à greffer les régions hypervariables d'une immunoglobuline murine appelée "région déterminant la complémentarité avec l'antigène" ou "CDR" sur les régions variables d'une immunoglobuline de classe G humaine.

Enfin, Clackson et al. (1991), Nature, 352**:** 624-628 et l'équipe de Lerner (Huse et al. (1989), Science, 246: 1275-1281) ont utilisé la technique PCR pour amplifier des gènes V des chaînes lourdes d'immunoglobulines G murines exprimées. Différents répertoires ont ainsi été créés à partir d'ADN génomique extrait de rate de souris immunisées. Une librairie de vecteurs portant les séquences des gènes variables de l'ensemble des anticorps a été réalisée. Un bactériophage λ est utilisé comme vecteur d'expression dans E. coli. Cette technique permet l'obtention de domaines VH simple chaîne des anticorps. On produit ensuite des fragments simple chaîne Fv, obtenus à partir d'un domaine VH, lié à un domaine VK par un linker, par utilisation de la technique PCR. Cette méthode permet donc de produire de façon simple et finalement peu coûteuse les fragments spécifiques dans un environnement non-immunogène.

Enfin, les anticorps monoclonaux utilisés selon l'invention, qu'ils soient de type IgG ou IgM, peuvent être mono-spécifiques, bi-spécifiques ou polyspécifiques. Les techniques de manipulation génétique décrites ci-dessus permettent d'associer dans une molécule des spécificités, d'une part, pour la ferritine et, d'autre part, pour un récepteur ou un antigène de surface spécifique d'une cellule que l'on souhaite cibler.

Il existe différentes possibilités pour utiliser les anticorps monoclonaux dans le concept de "magic bullet". La première est d'utiliser l'anticorps comme effecteur immunitaire, faisant appel dans les thérapies anti-tumorales à une activité de type CDCC (complément dependent cell cytotoxicity) ou ADCC (antibody dependent cell cytotoxicity). Il peut également s'agir d'un blocage d'un récepteur de la cellule tumorale ou d'une vaccination anti-idiotypique spécifique de l'antigène tumoral.

Dans l'utilisation selon l'invention, l'anticorps ou le fragment de celui-ci peut être couplé avec une molécule X, où X est une molécule toxique, un médicament ou une prodrogue ou une séquence nudéotidique ou un deuxième anticorps quelque soit sa spécificité de reconnaissance.

### a) X est une molécule toxique :

Un premier exemple est le cas où X est un isotope radioactif, de préférence à émissions β. Parmi les méthodes de couplage des isotopes radioactifs sur des protéines, notamment des anticorps monoclonaux, plusieurs possibilités existent. En effet, plusieurs travaux utilisant des anticorps polyclonaux antiferritines marqués à l'Indium 111 (in 111) et à Yttrium (Y 90) ont donné des résultats intéressants concernant, d'une part, l'imagerie des sites tumoraux, de préférence avec In 111 et, d'autre part, le traitement des formes réfractaires de la maladie de Hodgkin (Y 90) (P.E. Borchardt et al. (1998), The Journal of Nuclear Medicine, 39: n° 3, page 476).

Qu'il s'agisse d'IgM ou d'IgG, l'isotope radioactif est couplé à l'anticorps par des techniques de chélation décrites, soit dans Borchardt et al. (voir supra).

Le choix de l'Yttrium 90 comme radioélément thérapeutique est particulièrement pertinent : c'est un isotope à rayonnement β pur, sa demi-vie est de 67 heures et son rayon d'irradiation de 6,6 mn. Il semble parfaitement adapté au traitement des masses tumorales, notamment la maladie de Hodgkin, les lymphomes ou les cancers du pancréas notamment.

X peut également être une molécule toxique de type chaîne A de la ricine, l'Abrine ou de la toxine diphtérique. La toxicité de ces polypeptides est connue. Elles peuvent être couplées à l'immunoglobuline par des liaisons de type carboxylique ou par tout type de liaison permettant de coupler des protéines entre elles, notamment par leurs résidus -COOH, -NH2, -SH, etc., et qui sont bien connues de l'homme du métier.

### b) X est une molécule cytotoxique :

Des médicaments cytotoxiques sont connus, d'autres sont en développement. Ils ont comme propriété de bloquer la division cellulaire, soit par blocage de la transcription, soit par blocage de la traduction soit par induction d'apoptose. A titre d'exemple, on peut citer la mitomycine, l'adriamycine, le taxol, etc. Le problème qui subsiste toujours avec l'utilisation de tels médicaments est leur toxicité vis-à-vis de cellules saines, ou de tissus sains. Les anticorps monoclonaux selon l'invention, mono-spécifiques de la ferritine ou bi-spécifiques vis-à-vis de la ferritine et d'un récepteur permet le ciblage de ces substances sur les cellules choisies.

### c) X est un vecteur galénique ou une prodrogue.

Afin d'améliorer la stabilité et, éviter la biodégradation ou une activité toxique vis-à-vis des cellules saines, l'anticorps monoclonal selon l'invention peut être couplé à un vecteur de type liposome, ou des émulsions de type cationique déjà utilisées comme système d'administration de médicaments (Texeira et al. (1999) Pharmaceutical Research, 16: 30-36). Il peut s'agir également de lipides cationiques tels que ceux décrits par D. Deshpande et al. (1998), Pharmaceutical Research, 15: 1340-1347. Dans ce type de vecteurs, il est particulièrement approprié d'intégrer des molécules cytostatiques et/ou cytotoxiques de type ARN et ADN antisens. Néanmoins, pour les raisons citées plus haut et visant toutes à augmenter la spécificité du ciblage, et la diminution de la cytotoxicité des différents toxiques utilisables, les molécules citées au point b) ci-dessus peuvent être avantageusement portées par ces vecteurs.

### d) X est un deuxième anticorps,

- soit dirigé contre la ferritine permettant le cross-linkage de plusieurs molécules de ferritine,
- soit dirigé contre un antigène de cellules lymphoïdes dendritiques, macrophagiques ou de type Natural Killer (tels que CD20, CD30, CD33, HNK1, CD56, à titre d'exemples), permettant alors le recrutement de ladite cellule et son activation au niveau de la cellule tumorale,
- soit enfin dirigé contre un récepteur situé sur une cellule tumorale cible permettant ainsi le cross-linkage de la cellule cible avec la ferritine.

Dans les points a), b), c) et d) ci-dessus, X peut être également couplé à une chaîne carbonée sensible aux estérases. Cette chaîne, d'une longueur de 5 à 15 atomes de carbone, linéaire ou ramifiée, est sensible aux estérases. Le couplage de la molécule X à ce type de chaîne dans l'utilisation thérapeutique est particulièrement intéressant dans la mesure où les cellules saines ont des systèmes enzymatiques notamment des estérases, contrairement aux cellules cancéreuses. Donc, dans ce mode de réalisation, seules les cellules cancéreuses seront marquées par l'anticorps couplé à l'isotope marqué ; l'action des estérases dans les cellules saines conduira à un relargage de l'isotope dans la circulation générale, et donc seules les cellules cancéreuses bénéficient d'un marquage spécifique et différentiel.

La présente invention porte également sur un procédé de détermination ou de localisation de la présence éventuelle chez un individu d'une tumeur ou de cellules cancéreuses dans lesquelles le produit d'un gène de la famille c-myc, L-myc et N-myc est surexprimé comprend une étape de marquage in vitro, ex vivo ou in vivo desdites cellules ou de ladite tumeur avec un anticorps monoclonal anti-ferritine ou un fragment de celui-ci couplé directement ou indirectement à une substance émettrice d'un signal, ledit anticorps ou ledit fragment reconnaissant un épitope commun aux ferritines humaines acides et basiques. Dans ce procédé, l'anticorps est de préférence couplé à un isotope radioactif ; à titre d'exemple, l'Indium 111 est particulièrement approprié pour l'utilisation des anticorps monoclonaux selon l'invention en immunoscintigraphie. La qualité des anticorps monoclonaux selon l'invention telle que décrite plus haut, à savoir sa spécificité et son affinité, est importante dans le procédé de détermination ou de localisation selon l'invention.

L'homme du métier saura, en tant que de besoin; coupler à l'anticorps mono- ou bi-spécifique le signal approprié à la mise en évidence et/ou à la localisation d'une tumeur ou de cellules tumorales. A titre d'exemple, on peut citer les signaux émetteurs de fluorescence ou de luminescence couplés par toute méthode appropriée à l'anticorps. Un exemple de méthode largement utilisée est le couplage avidine/biotine.

Un autre objet de l'invention est le produit du couplage entre un anticorps monoclonal tel que décrit ci-dessus, reconnaissant un épitope commun aux ferritines humaines acides et basiques et ayant une affinité supérieure à 10⁻⁹ Mole/litre, avec une substance X choisie dans un groupe constitué de :
- radioisotopes à émission beta, alpha ou gamma ;
- molécules cytotoxiques de type chaîne A de la ricine, Abrine, ou chaîne A de la toxine diphtérique ;
- substances cytolytiques de type methotrexate, mitomycine, taxol
ou adriamycine ;
- des ARN et ADN antisens.

En particulier, l'épitope commun aux ferritines humaines acides et basiques est répétitif.

Elle concerne également les produits de couplage dans lesquels X est stabilisé par un vecteur de type liposome ou émulsion cationique. Elle concerne également un produit de couplage dans lequel un espaceur constitué d'une chaîne carboxylique entre 5 et 15 atomes de carbone est intégré dans le produit de couplage et permet de libérer la molécule X lorsque le produit de couplage est mis en présence d'estérases endogènes.

Dans l'ensemble des différents aspects de l'invention décrits ci-dessus, il était particulièrement surprenant de constater que l'utilisation des anticorps monoclonaux antiferritines ou fragments de ceux-ci, le cas échéant couplés à un isotope radioactif, à une substance cytotoxique ou cytostatique trouvait son application dans le diagnostic et le traitement de toutes les tumeurs et cellules cancéreuses qui présentent une surexpression du produit de gènes c-myc, L-myc et N-myc. Sans limiter pour autant le champ d'application, différents types de cancers pour lesquels ce type de traitement paraît particulièrement approprié sont les suivants :

### a) la maladie de Hodgkin :

Il était déjà connu et décrit que des patients réfractaires aux traitements classiques de la maladie ont pu être traités avec un anticorps polyclonal antiferritine marqué à l'Yttrium 90 (H.M. Vriesendorp et al (1997), Cancer Supplement, December 15, vol. 80, n° 12, pages 27-21).

### b) le cancer du pancréas :

Le cancer du pancréas concerne 8.000 nouveaux cas par an en France. Ce cancer a un pronostic catastrophique dans le sens où il y a moins de 3 % de survie à cinq ans. Ce cancer est peu chimio-sensibie ; il est radio-sensible mais radio-incurable (organe critique). Les traitements actuels sont la chirurgie, si possible, la radiothérapie ou la radiothérapie per-opératoire. L'avantage de l'utilisation des anticorps monoclonaux antiferritines couplés à un isotope radioactif, notamment de l'Yttrium 90 est que la dose de radiothérapie délivrable est potentiellement multipliée par 20, accompagnée d'une toxicité secondaire très faible. En effet, la conséquence du ciblage évite une destruction de l'isotope radioactif dans la circulation générale et ce dernier est directement ciblé à l'intérieur des tumeurs.

### c) le carcinome hépato-cellulaire :

Ce cancer du foie représente environ 15 cas pour 100.000 habitants. Ils sont opérables dans 20 % des cas avec un pronostic de 40 % à cinq ans. Dans les 80 % des cas inopérables, le pronostic est de 10% à cinq ans. Aucun traitement n'a encore prouvé son efficacité hormis le lipiodol radioactif. Le traitement par les composés de l'invention permet d'augmenter l'index thérapeutique (temps de résidence/émission) de 4 à 10 par rapport au traitement au lipiodol marqué à l'iode 131, également du fait de l'administration directement tumorale de l'isotope.

### d) le carcinome de la tête et du cou (cancer des voies aériennes digestives supérieures ou VADS).

Ces cancers concernent 12.000 cas par an en France et présentent 40 % de survie à cinq ans dont 15 % de ces survivants ont un risque élevé de formation métastasique.

Tous ces cancers cités ci-dessus produisent de la ferritine. En outre, alors que dans les cellules saines, la ferritine est exprimée seulement en intracellulaire, dans le processus néoplasique, reliée le plus souvent à l'oncogène c-myc, la ferritine s'exprime généralement sur la membrane cellulaire. Il apparaît ainsi clairement que de disposer du produit de couplage entre les anticorps monoclonaux et une molécule X telle que définie ci-dessus antiferritine permet de cibler spécifiquement un spectre varié de tumeurs ou de cellules tumorales, qui ont toutes en commun la spécificité de l'hyperexpression d'un gène de la famille myc. Dans le cas d'un traitement par radiothérapie métabolique, l'utilisation d'une IgM est intéressante de par sa diffusion lente et sa durée de vie plus élevée.

La liste des pathologies tumorales citées n'est pas exhaustive. Les neuroblastomes, les cancers des poumons ou de l'ovaire, présentent également une surexpression de c-myc.

Les exemples accompagnés des figures ci-dessous permettent d'illustrer l'invention et ses performances, sans pour autant la limiter.

Légende des figures :
La figure 1 représente des photographies de trois coupes histochimiques traitées comme indiqué ci-après et marquées à l'anticorps monoclonal antiferritine AMB8LK.
Les photographies A et B sont des coupes de ganglions de maladie de Hodgkin. La photographie C est une coupe d'adénocarcinome pancréatique.
La figure 2 représente le chromatogramme obtenu après purification sur Superdex 200 - Colonne 26/60 dans le protocole de purification décrit dans le tableau Il ci-après.
La figure 3 représente les résultats d'un test ELISA sandwich (AMB8 LK sur phase solide, AMB8 LK marqué à la phosphatase alcaline dans du sérum humain).

### Exemple 1 : obtention des anticorps monoclonaux antiferritines :

a) Comme il a été dit plus haut, il est important que l'antigène utilisé pour immuniser les cellules soit obtenu à un fort degré de pureté. La technique originale, utilisée est décrite dans le tableau II ci-dessous.

**Tableau II :**

| | |
|---|---|
| Rate humaine de Minkowsky-Chauffard | Découpage et broyage Sonication 20 min (Duty C. constant/G40 |
| Rate soniquée | Chauffage à 70 - 75° C pendant 10 min |
| Extrait de rate enrichi en ferritine | Centrifugation 25 000 g pendant 10 min |
| Surnageant de centrifugation | + acide acétique --- pH 4,8 Contact 2 heures à 4° C + K2HP04--- pH 6,5 |
| Surnageant de centrifugation | Précipitation fractionnée Sulfate d'ammonium 50 % 1 nuit 4°C Centrifugation 2 500 g pendant 10 min |
| Culot de centrifugation | Reprise en tampon K2HP04 100 mM, pH 7,2 Dialyse en tampon K2HP04 100 mM, pH 7,2 1 nuit 4° C |
| Surnageant de dialyse | Reprécipitation par du Sulfate de cadmium à 5% 1 nuit à 4°C Centrifugation à 10 000 g pendant 30 min |
| Culot de centrifugation | Reprise en tampon K2HP04 100 mM, pH 7,2 Dialyse en tampon K2HP04 100 mM, pH 7,2 1 nuit 4° C |
| Surnageant de dialyse | Chromatographie sur SUPERDEX 200 Concentration par ultrafiltration AMICON Tampon phosphate 100 mM, pH 7,2 |
| Ferritine 50 au sulfate de cadmium | Analyses physico-chimique et immunochimique (QC) |
| Standardisation et immunisation des souris | |

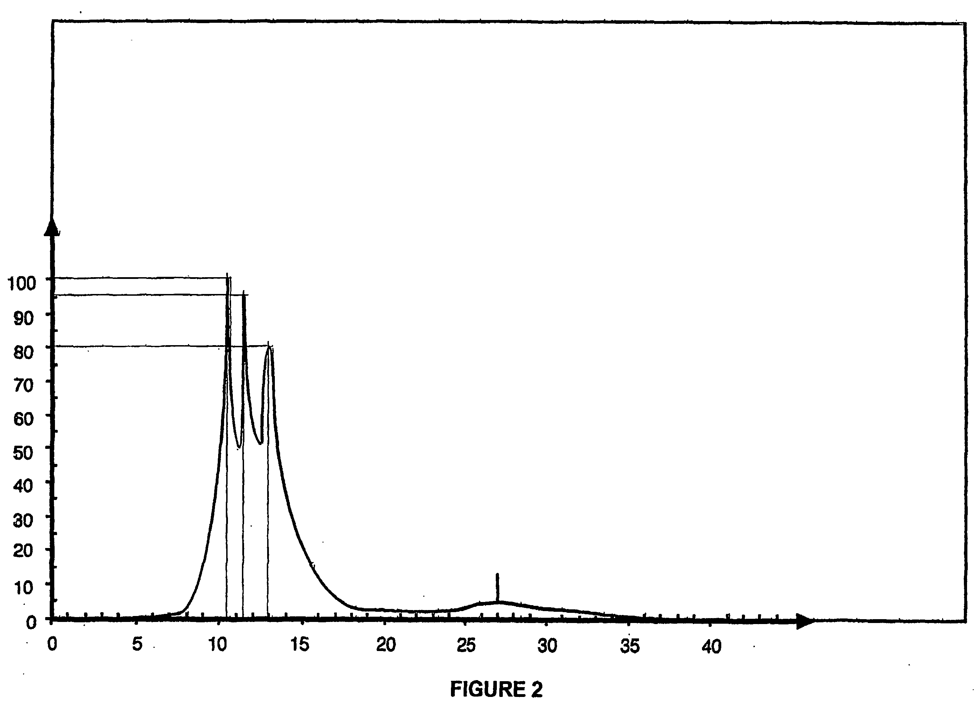

L'analyse du chromatogramme après chromatographie sur SUPERDEX 200 est représentée dans la figure 2.
b) immunisation des souris :
L'obtention et le clonage des anticorps monoclonaux par immunisation de souris sont réalisés par la méthode classique de Köhler et Milstein.

c) production et purification des anticorps monoclonaux produits par les hybridomes :

Les hybridomes sont mis en croissance dans un système de culture cellulaire in vitro sur multitrays (Nunc, références 16795).

Les cellules sont mises en culture dans du RPMI 4+ TCH. L'innoculum est constitué de 5 x 10⁷ dans 200 ml de TCH. Du milieu RPMI (environ 100 à 200 ml) est ajouté tous les deux jours jusqu'à confluence. Le surnageant est récolté après environ une semaine quand les cellules sont mortes.

Le surnageant est concentré dix fois puis chargé sur une colonne de chromatographie sur protéines G sépharose FF (Pharmacia), après dilution de moitié dans du tampon phosphate de sodium 20 mM pH 7. Après la chromatographie, l'anticorps est élué avec du tampon glycine HCl 0,1 M pH 7 et la colonne est dessalée immédiatement contre du tampon phosphate de potassium 0,2 M pH 7,2. Le pourcentage de pureté obtenu est d'au moins 95 %. L'isotype, le point isolélectrique et la réactivité par immunodiffusion sont ensuite étudiés sur ces préparations purifiées.

Parmi les différents anticorps obtenus et présentés dans le tableau I ci-dessus, les anticorps monoclonaux B8 et M29 apparaissent comme les plus intéressants en termes d'affinité et de spécificité pour les cellules humaines. Ces deux anticorps sont d'isotype IgG 1 Kapa.

### Résultats d'histochimie :

Les expériences en histochimie ont été réalisées avec un anticorps monoclonal B8 couplé à la peroxydase.
a) sur ganglion de Hodgkin,
b) sur adéno-carcinome pancréatique. Une étude d'immunohistochimie a été réalisée avec l'anticorps AMB8LK dilué au 1/250è en tampon PBS selon la technique classique à la phosphatase alcaline.

Les tissus ont été fixés par un bain de paraformaldehyde 4%, ethenolamide 0,1 M pH 7.4 et coulés dans la paraffine. Des coupes de 5 µ sont réalisées. Après déparaffinage par bains de xylène-ethanol, les réactions non spécifiques sont bloquées par incubation de la préparation avec de la sérum albumine bovine. La coupe est incubée avec l'anticorps AMB8LK à la dilution de 1/25è, une heure à température ambiante. La révélation de la fixation d'AMB8LK se fait par un système peroxydase (Cordell J.-L. et al, J. Histochem 1984 32: 219-229).

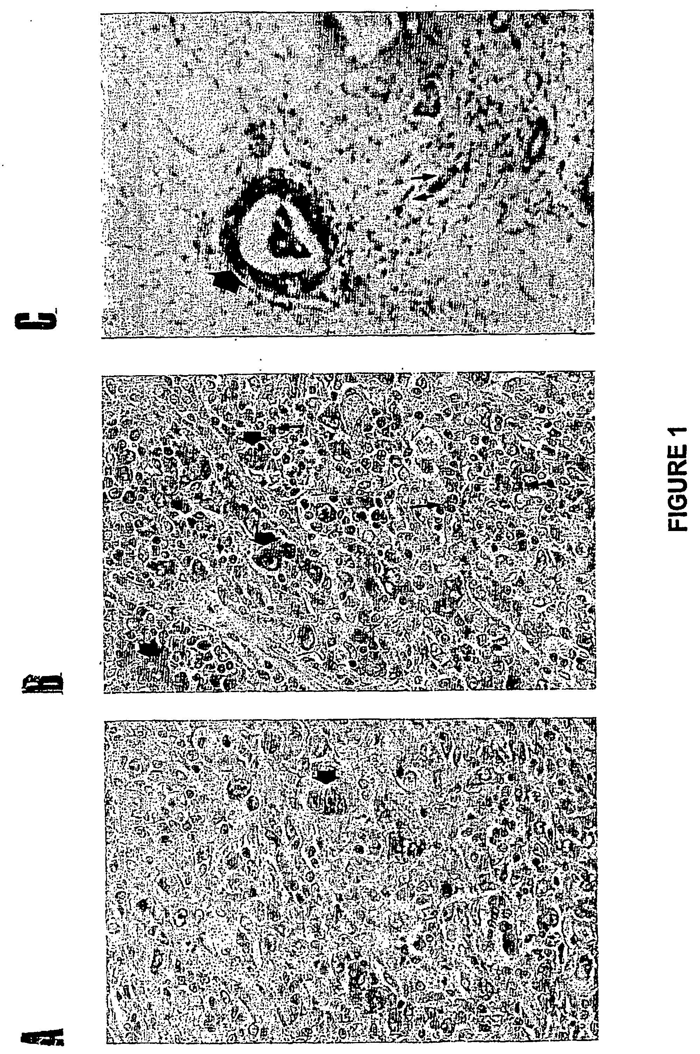

Les résultats obtenus sont représentés dans la figure 1.

L'observation des photographies A et B indique que toutes les cellules tumorales de Reed Sternberg sont positives pour l'expression de Ferritine.

L'observation de la photographie C indique également que la tumeur elle-même est fortement positive pour l'expression de la Ferritine.

### EXEMPLE 2 : Résultats d'un test ELISA Sandwich

La reconnaissance d'épitopes répétitifs communs aux isoferritines acides et basiques humaines permet d'améliorer les propriétés diagnostic et thérapeutiques des anticorps.

Le test sandwich ELISA permet d'identifier des anticorps dirigés contre des épitopes répétitifs. En effet, le principe du test repose sur la fixation d'un même antigène d'une part avec un premier anticorps fixé sur une phase solide et d'autre part avec un deuxième anticorps libre et portant un marqueur (enzymatique par exemple). Pour que le test soit fonctionnel, il faut que deux anticorps au moins puissent reconnaître une même molécule, autrement dit, que la molécule reconnue comprenne au moins deux épitopes répétés.

L'anticorps monoclonal de l'invention AMB8 LK a été utilisé pour la réalisation d'un test ELISA sandwich pour la détection des ferritines humaines.

Plus précisément, le test ELISA est réalisé selon le protocole suivant :

### a) Incubation :

50 µl de standard ou de sérum de patient sont déposés dans chaque puits. On ajoute 200 µl de l'anticorps anti-ferritine AMB8 LK conjugué à la péroxydase. On ajoute ensuite des billes coatées avec l'anticorps AMB8 LK dans chaque puits. Le mélange est incubé deux heures sous agitation à température ambiante. On lave ensuite 3 fois avec une solution de 1,5 ml de NaCl 9%. Tween 20 2%.

### b) Réaction colorée :

300 µl de susbstrat (OPD) est déposé dans chaque tube. Le mélange est incubé 30 minutes à température ambiante à l'abri de la lumière. On ajoute 2 ml d'HCl 1M dans chaque tube.

### c) Lecture :

On mesure l'absorbance à 492 nm qui reflète la quantité d'anticorps fixés à la ferritine, elle-même fixée aux anticorps de la phase solide.

La figure 3 présente les résultats du test.

Il permet la détection de la ferritine humaine d'origine :
- splénique
- hépatique
- cardiaque
- placentaire.

Les résultats présentés ici montrent qu'il est possible de réaliser un test sandwich avec l'anticorps AMB8 LK. Ces résultats démontrent que l'anticorps reconnaît un épitope commun répétitif sur les isoferritines humaines.

## Revendications

1. Utilisation, dans la fabrication d'un médicament pour la thérapie d'un cancer dont les cellules présentent une surexpression du produit d'un gène de la famille c-myc, L-myc et N-myc, d'un anticorps monoclonal anti-ferritine ou un fragment de celui-ci ou d'une construction incluant un tel fragment, ledit anticorps ou ledit fragment reconnaissant un épitope commun aux ferritines humaines acides et basiques:

2. Utilisation selon la revendication 1 dans laquelle ledit épitope commun aux ferritines humaines acides et basiques est répétitif.

3. Utilisation selon la revendication 1 ou la revendication 2 dans laquelle l'anticorps ou le fragment de celui-ci est couplé avec une molécule X, où un X est une molécule toxique, un médicament, une pro-drogue, ou un deuxième anticorps quelle que soit sa spécificité.

4. Utilisation selon l'une des revendications 1, 2 ou 3 dans laquelle l'anticorps monoclonal antiferritine est une IgG K d'affinité supérieure à 10⁻⁹ Mole/litre, ledit anticorps étant mono- ou bi-spécifique.

5. Utilisation selon la revendication 4 dans laquelle, quand l'anticorps est bi-spécifique, le premier paratope est afin pour la ferritine, et le deuxième paratope est affin pour un épitope spécifique d'un antigène de surface ou un récepteur spécifique d'une cellule cible.

6. Utilisation selon la revendication 1 , 2 ou 3 dans laquelle l'anticorps monoclonal est une IgM ou un fragment de cette-ci.

7. Utilisation selon la revendication 3 dans laquelle X est un radioisotope à émission beta.

8. Utilisation selon la revendication 3 dans laquelle X est une molécule toxique de type chaîne A de la ricine, Abrine, ou chaîne A de la toxine diphtérique.

9. Utilisation selon la revendication 3 dans laquelle X est une substance cytolytique de type methotrexate, mitomycine, taxol ou adriamycine.

10. Utilisation selon la revendication 3 dans laquelle X est une substance cytostatique et/ou cytotoxique de type ARN antisens.

11. Utilisation selon la revendication 3 dans laquelle X est une substance stabilisée par un vecteur de type liposome.

12. Utilisation selon la revendication 3 dans laquelle, X est un anticorps reconnaissant un récepteur spécifique d'une cellule tumorale.

13. Utilisation selon la revendication 3 dans laquelle X est un anticorps reconnaissant un antigène de cellules lympoïdes, dendritiques, macrophagiques ou de type Natural Killer.

14. Utilisation selon la revendication 3 dans laquelle X est un radioisotope à émission alpha.

15. Utilisation selon la revendication 3 dans laquelle X est un radioisotope à émission gamma.

16. Utilisation selon l'une des revendications 1 à 15 dans laquelle un espaceur C5 à C15 est intégré dans le produit de couplage et permet de libérer la molécule X lorsque le produit de couplage est mis en présence d'estérases.

17. Utilisation dans la fabrication d'un produit pour déterminer ou localiser la présence éventuelle chez un individu d'une tumeur ou de cellules cancéreuses dans lesquelles le produit de la famille d'un gène de la famille c-myc, L-myc et N-myc est surexprimé, d'un anticorps monoclonal anti-ferritine ou d'un fragment de celui-ci couplé directement ou indirectement à une substance émettrice d'un signal, ledit anticorps ou ledit fragment reconnaissant un épitope commun aux ferritines humaines acides et basiques.

18. Utilisation selon la revendication 17 dans laquelle ledit épitope commun aux ferritines humaines acides et basiques est répétitif.

19. Utilisation selon la revendication 17 ou 18 dans laquelle l'anticorps est couplé à un isotope radioactif à rayonnement beta, de type Yttrium-90, d'un rayonnement gamma de type Indium-111 ou d'un rayonnement alpha.

20. Utilisation selon la revendication 17 ou 18 dans laquelle l'anticorps est couplé à une substance émettrice d'un signal fluorescent ou luminescent.

21. Utilisation selon l'une des revendications 17 à 20 dans laquelle l'anticorps a une affinité supérieure à 10⁻⁹ Mole/litre, ledit anticorps étant mono- ou bi-spécifique.

22. Utilisation selon la revendication 21 dans laquelle, quand l'anticorps est bi-spécifique, le premier paratope est affin pour la ferritine, et le deuxième paratope est affin pour un épitope spécifique d'un antigène de surface ou un récepteur spécifique d'une cellule cible.

23. Produit de couplage entre un anticorps et une substance X choisie dans un groupe constitué de :
- radioisotopes à émission beta, alpha ou gamma ;
- molécules toxiques de type chaîne A de la ricine, Abrine, ou chaîne A de la toxine diphtérique ;
- substances cytolytiques de type methotrexate, mitomycine, taxol ou adriamycine ;
- des ARN antisens.
ledit anticorps étant un anticorps monoclonal anti-ferritine reconnaissant un épitope commun aux ferritines humaines acides et basiques et ayant une affinité pour l'antigène supérieure à 10⁻⁹ Mole/litre.

24. Produit de couplage selon la revendication 23 dans lequel ledit épitope commun aux ferritines humaines acides et basiques est répétitif.

25. Produit de couplage selon la revendication 23 dans lequel X est stabilisé par un vecteur de type liposome.

26. Produit de couplage selon la revendication 23 dans lequel un espaceur C5 à C15 est intégré dans le produit de couplage et permet de libérer la molécule X lorsque le produit de couplage est mis en présence d'estérases.

## Patentansprüche

1. Verwendung eines monoklonalen Anti-Ferritin-Antikörpers oder eines Fragments davon oder einer Konstruktion, die ein solches Fragment enthält, wobei der Antikörper oder das Fragment ein Epitop erkennen, das den sauren und basischen Ferritinen des Menschen gemein ist, bei der Herstellung eines Medikaments für die Therapie eines Krebses, dessen Zellen eine Überexpression des Produkts eines Gens der Familie c-myc, L-myc und N-myc aufweisen.

2. Verwendung nach Anspruch 1, bei der das Epitop, das den sauren und basischen Ferritinen des Menschen gemein ist, repetitiv ist.

3. Verwendung nach Anspruch 1 oder Anspruch 2, bei der der Antikörper oder das Fragment davon mit einem Molekül X gekoppelt ist, wo ein X je nach seiner Spezifizität ein toxisches Molekül, ein Medikament, ein Pro-Drug oder ein zweiter Antikörper ist.

4. Verwendung nach einem der Ansprüche 1, 2 oder 3, bei der der monoklonale Anti-Ferritin-Antikörper ein IgG K mit Affinität über 10⁻⁹ Mol/Liter ist, wobei der Antikörper mono- oder bispezifisch ist.

5. Verwendung nach Anspruch 4, bei der, wenn der Antikörper bispezifisch ist, das erste Paratop für das Ferritin affin ist und das zweite Paratop für ein spezifisches Epitop eines Oberflächenantigens oder einen spezifischen Rezeptor einer Zielzelle affin ist.

6. Verwendung nach Anspruch 1, 2 oder 3, bei der der monoklonale Antikörper ein IgM oder ein Fragment davon ist.

7. Verwendung nach Anspruch 3, bei der X ein Radioisotop mit Betaemission ist.

8. Verwendung nach Anspruch 3, bei der X ein toxisches Molekül vom Typ A-Kette von Ricin, Abrin oder A-Kette von Diphterietoxin ist.

9. Verwendung nach Anspruch 3, bei der X eine cytolytische Substanz vom Typ Methotrexat, Mitomycin, Taxol oder Adriamycin ist.

10. Verwendung nach Anspruch 3, bei der X eine cytostatische und/oder cytotoxische Substanz vom Typ Antisens-RNA ist.

11. Verwendung nach Anspruch 3, bei der X eine durch einen Vektor vom Typ Liposom stabilisierte Substanz ist.

12. Verwendung nach Anspruch 3, bei der X ein Antikörper ist, der einen spezifischen Rezeptor einer Tumorzelle erkennt.

13. Verwendung nach Anspruch 3, bei der X ein Antikörper ist, der ein Antigen von lymphatischen Zellen, Dendritenzellen, Makrophagenzellen oder vom Typ Natural Killer erkennt.

14. Verwendung nach Anspruch 3, bei der X ein Radioisotop mit Alphaemission ist.

15. Verwendung nach Anspruch 3, bei der X ein Radioisotop mit Gammaemission ist.

16. Verwendung nach einem der Ansprüche 1 bis 15, bei der ein Spacer C5 bis C15 in das Kopplungsprodukt integriert ist und ermöglicht, das Molekül X freizusetzen, wenn des Kopplungsprodukt mit Esterasen zusammengebracht wird.

17. Verwendung eines monoklonalen Anti-Ferritin-Antikörpers oder eines Fragments davon direkt oder indirekt gekoppelt an eine Substanz, die ein Signal emittiert, wobei der Antikörper oder das Fragment ein Epitop erkennt, das den sauren und basischen Ferritinen des Menschen gemein ist, bei der Herstellung eines Produkts zur Bestimmung oder Lokalisierung des eventuellen Vorhandenseins eines Tumors oder von Krebszellen bei einer Person, bei denen das Produkt der Familie eines Gens der Familie c-myc, L-myc und N-myc überexprimiert ist.

18. Verwendung nach Anspruch 17, bei der das Epitop, das den sauren und basischen Ferritinen des Menschen gemein ist, repetitiv ist.

19. Verwendung nach Anspruch 17 oder 18, bei der der Antikörper an ein radioaktives Isotop mit Betastrahlung vom Typ Yttrium-90, mit Gammastrahlung vom Typ Indium-111 oder mit Alphastrahlung gekoppelt ist.

20. Verwendung nach Anspruch 17 oder 18, bei der der Antikörper an eine Substanz gekoppelt ist, die ein fluoreszentes oder lumineszentes Signal emittiert.

21. Verwendung nach einem der Ansprüche 17 bis 20, bei der der Antikörper eine Affinität über 10⁻⁹ Mol/Liter aufweist, wobei der Antikörper mono- oder bispezifisch ist.

22. Verwendung nach Anspruch 21, bei der, wenn der Antikörper bispezifisch ist, das erste Paratop für das Ferritin affin ist und das zweite Paratop für ein spezifisches Epitop eines Oberflächenantigens oder einen spezifischen Rezeptor einer Zielzelle affin ist.

23. Kopplungsprodukt zwischen einem Antikörper und einer Substanz X ausgewählt aus der Gruppe gebildet aus:
- Radioisotopen mit Betaemission, Alphaemission oder Gammaemission;
- toxischen Molekülen vom Typ A-Kette von Ricin, Abrin oder A-Kette des Diphterietoxins;
- cytolytischen Substanzen vom Typ Methotrexat, Mitomycin, Taxol oder Adriamycin;
- Antisens-RNAs,
wobei der Antikörper ein monoklonaler Anti-Ferritin-Antikörper ist, der ein Epitop erkennt, das den sauren und basischen Ferritinen des Menschen gemein ist, und eine Affinität für das Antigen über 10⁻⁹ Mol/Liter aufweist.

24. Kopplungsprodukt nach Anspruch 23, bei dem das Epitop, das den sauren und basischen Ferritinen des Menschen gemein ist, repetitiv ist.

25. Kopplungsprodukt nach Anspruch 23, bei dem X durch einen Vektor vom Typ Liposom stabilisiert ist.

26. Kopplungsprodukt nach Anspruch 23, bei dem ein Spacer C5 bis C15 in das Kopplungsprodukt integriert ist und ermöglicht, das Molekül X freizusetzen, wenn des Kopplungsprodukt mit Esterasen zusammengebracht wird.

## Claims

1. Use, in the manufacturing of a therapeutic drug for a cancer, the cells of which exhibit an overexpression of the product of a gene from the c-myc, L-myc and N-myc family, of an antiferritin monoclonal antibody or of a fragment thereof or of a construction including such a fragment, said antibody or said fragment recognizing an epitope that is common to acidic and basic human ferritins.

2. Use according to claim 1, in which said epitope that is common to acidic and basic human ferritins is repetitive.

3. Use according to claim 1 or claim 2, in which the antibody or fragment thereof is coupled to a molecule X, where X is a toxic molecule, a drug, a prodrug, or a second antibody regardless of its specificity.

4. Use according to any one of claim 1, 2 or 3 in which the antiferritin monoclonal antibody is an IgG K with an affinity of more than 10⁻⁹ Mole/liter, said antibody being mono- or bi-specific.

5. Use according to claim 4 in which, when the antibody is bi-specific, the first paratope has an affinity for ferritin, and the second paratope has an affinity for a specific epitope of a surface antigen or a specific receptor of a target cell.

6. Use according to claim 1, claim 2 or claim 3, in which the monoclonal antibody is an IgM or a fragment thereof.

7. Use according to claim 3, in which X is a beta-emitting radioisotope.

8. Use according to claim 3, in which X is a toxic molecule of the A chain ricin or abrin type, or A chain diphtheria toxin.

9. Use according to claim 3, in which X is a cytolytic substance of the methotrexate, mitomycin, taxol or adriamycin type.

10. Use according to claim 3, in which X is a cytostatic and/or cytotoxic antisense RNA.

11. Use according to claim 3, in which X is a substance stabilised by a liposome type vector.

12. Use according to claim 3, in which X is an antibody recognizing a specific receptor of a tumor cell.

13. Use according to claim 3, in which X is an antibody recognizing an antigen of lymphoid, dendritic, macrophagic or natural killer type cells.

14. Use according to claim 3, in which X is an alpha-emitting radioisotope.

15. Use according to claim 3, in which X is a gamma-emitting radioisotope.

16. Use according to any one of claims 1 to 15, in which a C5 to C 15 linker is integrated into the coupling product and can release the X molecule when the coupling product is brought into the presence of esterases.

17. Use, in the manufacturing of a product to determine or localise in an individual the presence of a tumor or cancerous cells in which the product of a gene from the c-myc, L-myc and N-myc family is overexpressed, of an antiferritin monoclonal antibody or a fragment thereof, directly or indirectly coupled to a signal-emitting substance, said antibody or said fragment recognizing an epitope that is common to acidic and basic human ferritins.

18. Use according to claim 17, in which said epitope that is common to acidic and basic human ferritins is repetitive.

19. Use according, to claim 17 or claim 18, in which the antibody is coupled to a beta-emitting yttrium-90 type, a gamma-emitting indium-111 type or an alpha-emitting radioactive isotope.

20. Use according to claim 17 or claim 18, in which the antibody is coupled to a substance emitting a fluorescent or luminescent signal.

21. Use according to any one of claims 17 to 20, in which the antibody has an affinity of more than 10⁻⁹ Mole/liter, said antibody being mono- or bi-specific.

22. Use according to claim 21 in which, when the antibody is bi-specific, the first paratope has an affinity for ferritin, and the second paratope has an affinity for a specific epitope of a surface antigen or a specific receptor of a target cell.

23. A coupling product between an antibody and a substance X selected from the group formed by:
• Beta-, alpha- or gamma-emitting radioisotopes;
• toxic A chain ricin or abrin type molecules, or A chain diphtheria toxin;
• cytolytic substances of the methotrexate, mitomycin, taxol or adriamycin type;
• antisense RNAs;
said antibody being an antiferritin monoclonal antibody recognizing an epitope that is common to acidic and basic human ferritins and having an affinity for the antigen of more than 10⁻⁹ Mole/liter.

24. A coupling product according to claim 23, in which said epitope that is common to acidic and basic human ferritins is repetitive.

25. A coupling product according to claim 23, in which X is stabilised by a liposome type vector.

26. A coupling product according to claim 23, in which a C5 to C15 linker is integrated into the coupling product and can release the X molecule when the coupling product is brought into the presence of esterases.
